# EUROPEAN PATENT SPECIFICATION

(11) **EP 3 791 909 B1**
(45) Date of publication and mention of the grant of the patent: **14.06.2023**
(21) Application number: 20206520.7
(22) Date of filing: 16.06.2011
(51) Int. Cl.: A61M 5/315

(54) **ADJUSTABLE PLUNGER DOSE STOP**
VERSTELLBARER STÖSSELDOSISSTOPP
ARRÊT DE DOSE DE PISTON RÉGLABLE

(30) Priority: 18.06.2010 US 34425610 P
(43) Date of publication of application: 17.03.2021
(62) Divisional of application: 11170245.2
(73) Proprietor: Becton, Dickinson and Company, Franklin Lakes, NJ 07417-1880 (US)
(72) Inventor: GOLD, Howard, Randolph, NJ 07869 (US)
(74) Representative: dompatent von Kreisler Selting Werner - Partnerschaft von Patent- und Rechtsanwälten mbB

(56) References cited:
- WO-A2-02/26298
- US-A- 3 327 904
- US-B1- 6 569 126

## Description

### Field of the Invention

The present invention relates generally to an adjustable plunger dose stop to control dosage. More particularly, the present invention relates to an adjustable plunger dose stop for a syringe. Still more particularly, the present invention relates to a syringe having an adjustable collar that limits travel of the plunger when drawing a dose, thereby controlling dosage.

### Background of the Invention

Users of existing syringes generally must visually measure the liquid medication level being drawn from a container into the syringe using scale markings while the dose is being drawn. A need exists for a syringe in which the user does not have to continuously visually monitor the scale markings while drawing a dose.

The amount of a dose is determined by the volume of liquid medication drawn into the syringe. The scale markings run perpendicular to the longitudinal axis of the syringe. The scale markings can be difficult to read making drawing an accurate dose difficult. Accordingly, a need exists for a syringe in which the dose setting is easily viewed and determined.

Furthermore, the user determines the amount of liquid medication drawn into the syringe. Thus, when the user is not being careful, too little or too much medication can be drawn into the syringe. Accordingly, a need exists for a syringe in which an accurate dose is easily drawn into the syringe. In US 3 327 904 a syringe is described for dispensing and drawing in medication according to the preamble of claim 1.

### Summary of the Invention

The present invention is defined by claim 1 wherein a syringe has an adjustable collar that limits travel of the plunger when drawing a dose, thereby controlling dosage.

A syringe having an adjustable plunger dose stop allows a user to select the desired dose prior to drawing liquid medication from a container and provides a mechanical stop that limits the amount of liquid medication drawn into the syringe by the plunger. Additionally, the user is not required to visually measure the liquid medication using scale markings as required when using existing syringes.

Several advantages are achieved by eliminating the need for scale marking on the syringe. The syringe barrel can be shorter and wider, thereby allowing the overall length of the syringe to be shortened. Only the selected dose setting number can be visible to the user, thereby providing an easy-to-use syringe. The dose setting number may be oriented along the axis of the syringe, thereby providing easier and improved viewing of the dose setting.

The foregoing objectives are obtained by a syringe including a body for receiving a medicament and a collar rotatably disposed in the body. The collar is rotatable to set a desired dose. A plunger is movably disposed in the collar. The plunger is movable to draw in and dispense the liquid medicament.

The foregoing objectives are also obtained by a syringe including a body having a first portion and a second portion. The second portion receives a liquid medicament. A collar is rotatably disposed in or on the body. The collar is rotatable to set a desired dose. A plunger rod is movably connected to the collar. A stopper is disposed at an end of the plunger rod to facilitate drawing in and dispensing the liquid medicament.

The foregoing objectives are also obtained by a method of injecting a liquid medicament with a syringe. A collar is rotated with respect to a syringe body in which the collar is disposed to set a desired dose. A plunger is moved axially in a first direction with respect to the collar to draw the liquid medicament into the syringe body. The plunger is moved axially in a second direction with respect to the collar to dispense the liquid medicament from the syringe body.

Objects, advantages, and salient features of the invention will become apparent from the following detailed description, which, taken in conjunction with the annexed drawings, discloses exemplary embodiments of the invention.

As used in this application, the terms "front," "rear," "upper," "lower," "upwardly," "downwardly," and other orientational descriptors are intended to facilitate the description of the exemplary embodiments of the present invention, and are not intended to limit the structure thereof to any particular position or orientation.

### Brief Description of the Drawings

The above benefits and other advantages of the various embodiments of the present invention will be more apparent from the following detailed description of exemplary embodiments of the present invention and from the accompanying drawing figures, in which:
FIG. 1 is a perspective view of a syringe in accordance with an exemplary embodiment of the present invention;
FIG. 2 is elevational view of a collar and plunger assembly of the syringe of FIG. 1;
FIG. 3 is an elevational view of a syringe body in accordance with the syringe of FIG. 1;
FIG. 4 is a rear elevational view of the collar nut of FIG. 2;
FIG. 5 is an elevational view of an assembled syringe in accordance with an exemplary embodiment of the present invention including the collar and plunger assembly of FIG. 2 and the syringe body of FIG. 3;
FIG. 6 is an elevational view in partial cross section of a syringe example not being part of the present invention:
FIG. 7 is a perspective view of an example not being part of the invention in which a sleeve having a cutout therein is disposed on an outer surface of the syringe body;
FIG. 8 is an elevational view of a collar having the dose numbers disposed on an outer surface thereof;
FIG. 9 is an elevational view of a syringe body having the dose numbers disposed on an outer surface thereof;
FIG. 10 is an elevational view of a collar having an external thread groove;
FIG. 11 is an elevational view of a syringe body having a positive internal thread groove for receiving the collar of FIG. 10;
FIG. 12 is an elevational view of a collar having a positive external thread groove;
FIG. 13 is an elevational view of a syringe body having an internal thread groove for receiving the collar of FIG. 12;
FIG. 14 is an elevational view in partial cross section of a collar having a protrusion to threadably engage thread grooves of a syringe body;
FIG. 15 is an elevational view of a syringe body having a protrusion to threadably receive thread grooves of a collar;
FIG. 16 is an elevational view of a sleeve disposed on a syringe body;
FIG. 17 is an elevational view of a collar having a plurality of ribs disposed on the handle;
FIG. 18 is an elevational view of a collar having a cylinder extending therefrom to receive a sterility cap;
FIG. 19 is an elevational view of a sterility cap connected to a collar;
FIG. 20 is an elevational view of a sterility cap having a hexagonal shaped handle without a cylinder extending therefrom;
FIG. 21 is a perspective view of a syringe in accordance with another exemplary embodiment of the present invention;
FIG. 22 is a perspective view in cross section of the syringe of FIG. 21;
FIG. 23 is a perspective view of a syringe body of the syringe of FIG. 21; and
FIG. 24 is a perspective view of a collar of the syringe of FIG. 21.

Throughout the drawings, like reference numbers will be understood to refer to like parts, components and structures.

### Detailed Description of Exemplary Embodiments

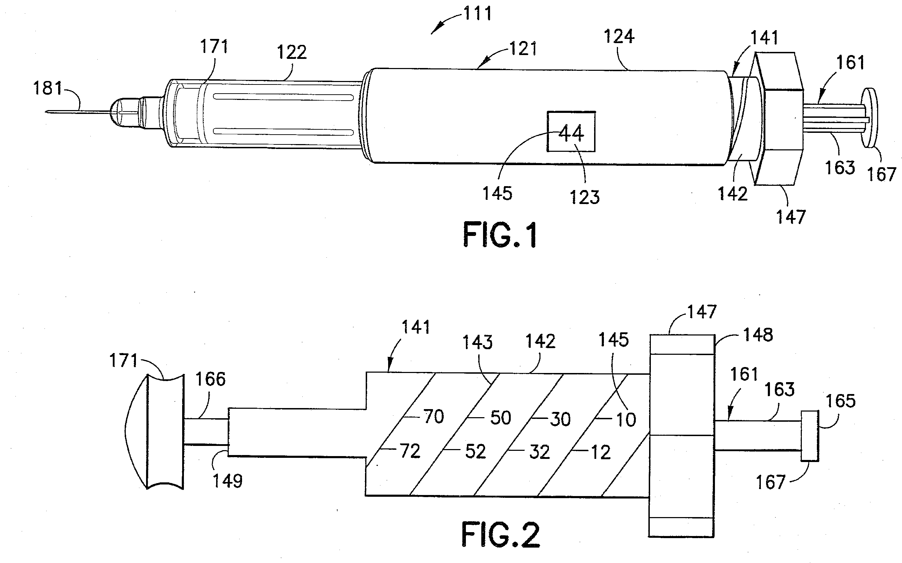

In an exemplary embodiment of the present invention, as shown in FIGS. 1 - 5, a syringe 111 includes a syringe body 121, a collar 141 rotationally and axially movable with respect to the syringe body 121, and a plunger 161 movable with respect to the collar 141. All of these components are preferably made from suitable plastic material(s). A metal needle 181 is connected to the syringe body 121. Liquid medication is drawn from a container (not shown) through the needle 181 into the syringe body 121 and injected from the syringe body through the needle into an injection site. The exemplary embodiments of the present invention are applicable to any type of syringe, such as an insulin syringe or other type of syringe, such as for dispending growth hormone or anesthesia. The syringe may have an integral barrel to provide a permanently attached needle or may have a threaded, snap-fit or other type of barrel design to provide a removable needle. The needle 181 may also be deleted if desired.

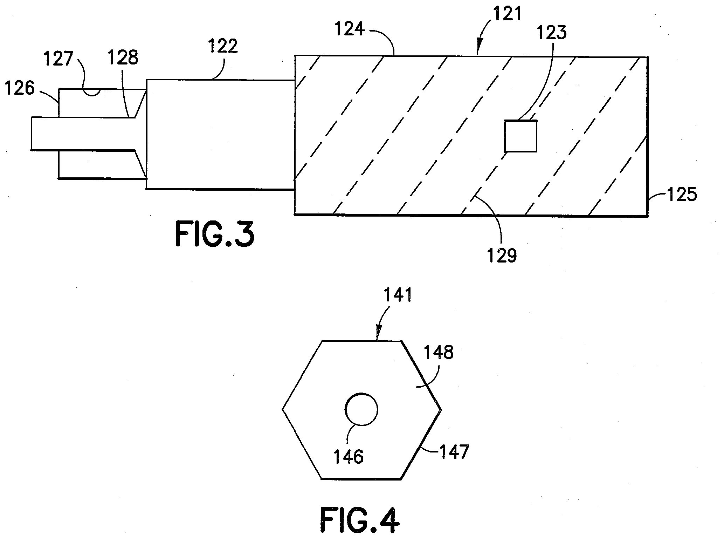

The syringe 111 has a window 123 through which the dose setting is displayed, preferably in large numbers that are easy for a user to see, learn and use, as shown in FIGS. 1 and 3. The user dials the desired dose and then draws up the liquid medicament by pulling the plunger 161 out of the syringe body 121 until the plunger abuts a mechanical stop, such as collar 141, thereby limiting the amount of medication drawn into the syringe to the set dose. In existing syringes, the plunger is withdrawn until a stopper is aligned with scale markings, which correspond to dose settings, that are disposed on the syringe body in a direction perpendicular to the withdrawal direction of the plunger.

The collar 141 is rotationally and axially movable with respect to the syringe body 121, as shown in FIGS. 1 and 2. Threads 143 are disposed on an outer surface 142 of the collar 141. A bore 146 passes through the collar 141 from a first end 148 to a second end 149 to receive the plunger rod 163 therethrough. The outer surface 142 of the collar 141 has a plurality of indicators 145, which correspond to the dose setting, disposed proximal the threads 143. The indicators 145 may be in fractions of a unit, single units or multiple units. The indicators may be numerals, symbols or Braille to facilitate use by users having impairments. Alternatively, the indicators may be displayed digitally. A handle 147 is disposed at the first end 148 of the collar 141 to facilitate rotation of the collar 141 by a user to set the dose. The second end 149 of the collar 141 has a smaller diameter than the first end 148 and acts as a mechanical stop for the plunger 161 when pulling the plunger out of the collar 141 to draw medication from a container into the syringe body 121, thereby limiting the amount of medication drawn into the syringe to the set dose.

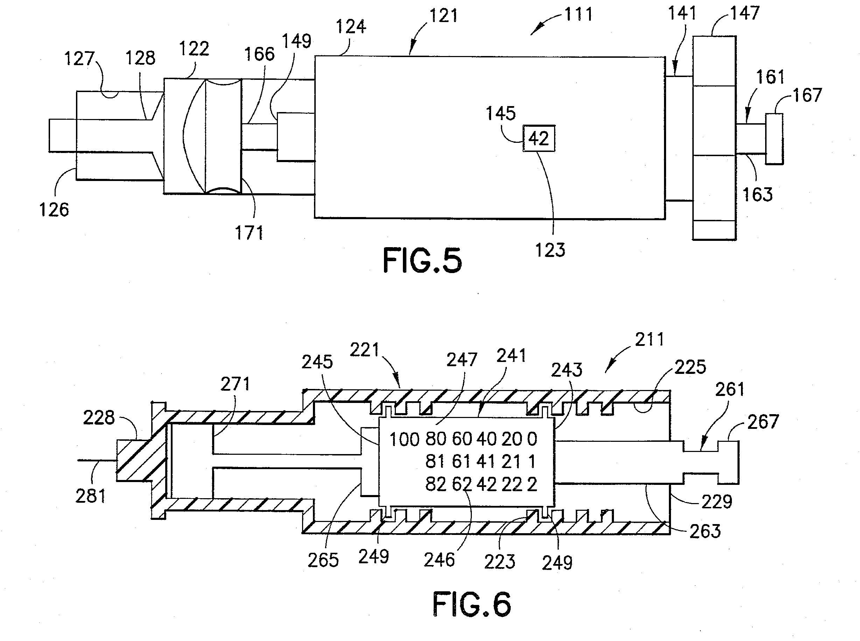

Preferably, the collar bore 146 and plunger rod 163 are substantially circular such that rotation of the plunger rod 163 does not result in undesired rotation of the collar 141, which could affect the set dose. Accordingly, the plunger rod 163 is free to rotate in the bore 146 without resulting in rotation of the collar 141, i.e., the plunger 161 moves independently of the collar 141. Thus, when a user rotates the plunger to draw in or inject liquid medication, the collar does not rotate with the plunger and affect the dose setting. Alternatively, the bore 146 is keyed to prevent rotational movement of the plunger rod 163 with respect to the collar 141. For example, the collar bore 146 and the plunger rod 123 may be substantially hexagonally-shaped, although any suitable shape may be used. Thus, the keyed plunger rod 163 rotates with rotation of the collar 141, and the keyed plunger rod 163 is axially movable without moving the collar 141. For example, as shown in FIG. 6, the plunger 261 is keyed to the collar 241 such that the plunger handle 247 acts as the collar handle. Rotation of the plunger handle 267 results in rotation of the collar 241 with respect to the syringe 211 without resulting in axial movement of the plunger 261. The plunger handle 267 is rotated until the desired dose is set.

The syringe body 121 has a first portion 122 and a second portion 124, as shown in FIGS. 1 and 3. The first portion 122 receives liquid medicament and the second portion receives the collar 141, as shown in FIG. 1. Preferably, the first and second portions are integrally formed such that the syringe body is a single, unitary member. Alternatively, the first portion 122 may be connected to the second portion 124 such that after performing an injection the first portion may be removed and properly disposed of while retaining the second portion for further use. The first portion 122 may be removably connected to the second portion 124 in any suitable manner, including, but not limited to, locking tabs, snaps, threads or a grooved release feature that is retained by an O-ring or ball bearing housing on the second portion 124. The seal 171 has a neck that extends along the length of the disposable first portion 122 to prevent the second portion from contacting the fluid path. A stopper or seal 171 has an internal feature that accepts the tip of the plunger rod 163, as shown in FIG. 2, and can be assembled and disassembled. Alternatively, the seal 271 and plunger rod 263 can be unitarily formed as a single plastic piece, as in the plunger 261 of FIG. 6. The first portion 122 has a wall at one end that prevents the seal from coming out of the syringe body when the disposable tip is disassembled from the first portion 122 and the plunger. A diameter of the second portion 124 is larger than a diameter of the first portion 122, as shown in FIGS. 1 and 3.

The syringe can be an integrated unit having a shield at the needle end of the disposable first portion 122 that protects the needle and includes seal rings that provide a sterility barrier. When the disposable first portion 122 is contained in packaging that provides a sterility barrier then the shield may or may not provide a sterility barrier. The disposable first portion is contained in a cover that is closed with a cap or label that is sealed and provides the sterility barrier. Alternatively, the seal may be disposed at the end of the medicine containing housing and provides the sterility barrier.

In an example not being part of the invention, the syringe body may be of a single diameter over its entire length and the collar may be of a single diameter over its entire length.

The first portion 122 of the syringe body 121 is preferably transparent so that the medicine is visible to the user and following an injection the user can verify that the medicine has been injected. The second portion 124 of the syringe body 121 has a window 123 that exposes the desired dose setting. The second portion 124 of the syringe body 121 may be of a non-transparent color material and the window 123 is formed by the absence of material. Alternatively, the window 123 is formed by making the surface of the second portion 124 non-transparent except for a window 123 that exposes a single dose setting. Alternatively, a label having a cutout window is disposed on the second portion 124 of the syringe body 121. Alternatively, a band of ink is applied to the second portion 124 that exposes only the desired dose setting. Alternatively, the window is positioned at an end of the syringe body 121 such that a cutout is formed by only three sides of the window.

The plunger may have an integral stop feature, such as flange 265 of FIG. 6, that prevents the plunger from traveling past the collar and opens the syringe housing to the proper volume to provide the set dose. Alternatively, the seal bottoms out against the collar, as shown in FIGS. 2 and 5, that regulates the amount of medicine drawn.

The plunger can move independently of the collar. Alternatively, the plunger may be keyed to the collar so that the plunger handle serves as the collar handle.

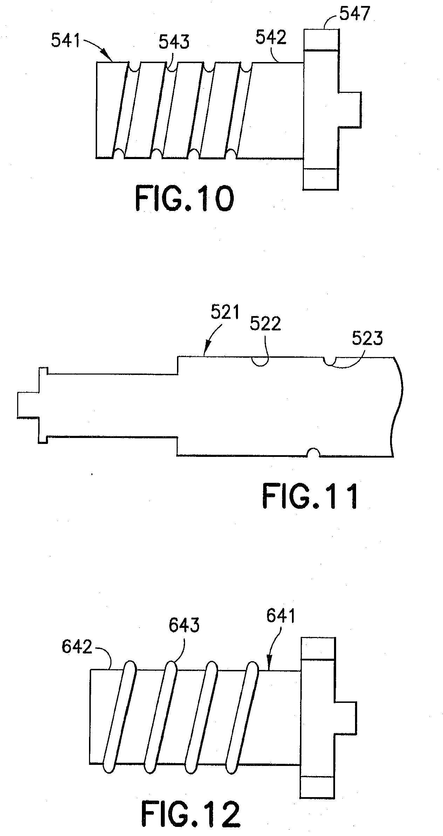

Internal threads 129 are disposed on an inner surface of the second portion 124 of the syringe body 121, as shown in FIG. 3, and engage the threads 143 on the outer surface 142 of the collar 141 for rotational and axial movement of the collar with respect to the syringe body 121. The threads 129 can extend along a portion of the inner surface of the syringe body 121 as shown in FIG. 3, extend along the entire inner surface of the syringe body, or a single notch can be used that receives the threads 143 of the collar 141 as shown in FIG. 11. A window 123 in the second portion 124 of the syringe body 121 allows a user to view the dose setting indicators 145 as the collar 141 is rotated out of the syringe body. The dose setting indicator 145 viewable in the window 123 corresponds to the dose setting. The second portion 124 of the syringe body 121 may be opaque, marked with ink, or any other suitable means may be used such that only the dose setting indicator viewable through the window 123 is visible. For example, the second portion 124 of the syringe body 121 may be textured such that only the dose setting indicator positioned in the window 123 is viewable.

An inner surface 127 of the syringe body proximal a second end 126 thereof 121 has internal threads (not shown) to receive a needle hub assembly, such as by a luer lock. Alternatively, a needle may be connected to the second end 126 of the syringe body 121 during manufacturing. Accordingly, the needle may be permanently attached or detachable, such as by a luer lock. As a further alternative, the syringe may be used without a needle, as for example to administer a fluid through an intravenous line via a needleless valve, port or injection site.

The plunger 161 includes a plunger rod 163 axially movable with respect to the collar 141. A first end 165 of the plunger rod 163 has a plunger head 167 disposed externally of the collar 141. The plunger head 167 is larger than the collar bore 146 to prevent the first end 165 of the plunger from entering the bore. A second end 166 of the plunger rod 163 is disposed externally of the collar 141 and within the syringe body 121.

The separate rubber seal, or stopper, 171 is disposed at a second end 166 of the plunger 161. The seal 171 is movable within the syringe body 121 between a first position at a reduced diameter portion 128 of the syringe body and a second position at the mechanical stop at the second end 149 of the collar 141. The seal 171 creates a fluid seal within the syringe body 121 that prevents liquid medication from passing beyond the seal.

A guard member may be provided at the needle end of the syringe that is movable along an axis of the syringe. The guard member is adjustable to expose a variable needle length as required. For example, the guard member may be fully retracted to expose the entire needle length to draw medication from a container. When the medication is delivered, the guard member can be adjusted to a position in which less of the needle is exposed. After the medication has been delivered, the guard member can be moved to a position in which the entire needle is covered, thereby providing a safety shield to prevent accidental needle sticks.

To perform an injection, the desired dose to be injected is dialed in with the adjustable collar 141. Initially, the seal 171 is in the first position engaging the reduced diameter portion 128 of the syringe body. The collar handle 147 is rotated, thereby axially moving the collar 141 out of the syringe body 121. When the dose indicator 145 corresponding to the desired dose setting appears in the window 123, the desired dose is ready to be drawn into the first portion 122 of the syringe body 121. The larger the dose to be delivered, the further the collar 141 moves out of the syringe body 121 and the farther from the reduced diameter portion 128 of the syringe body 121 the second end 149 of the collar 141 moves. The plunger 161 can be pulled rearwardly to draw air into the syringe body 121.

The needle 181 is inserted (typically through a septum) into a container, such as an insulin vial, in which the liquid medication to be delivered is stored. When the plunger 161 has been drawn rearwardly to draw air into the syringe body 121, the plunger 161 is pushed inwardly to expel the air and seat the seal 171 against the reduced diameter portion 128 of the syringe body 121. The plunger head 167 is drawn out of the collar 141, thereby moving the seal 171 rearwardly, until the seal 171 abuts the second end 149 of the collar 141, thereby limiting further movement of the plunger 161. Preferably, a locking means (not shown) prevents rotation of the collar 141 while the plunger 161 is drawn outwardly, thereby preventing accidental changes to the set dose. The amount of medication drawn into the syringe body 121 corresponds to the dose setting visible in the window 123.

To inject the drawn dose, the plunger 161 is pushed back into the collar 141 until the seal 171 returns to the first position abutting the reduced diameter portion 128 of the syringe body 121, thereby preventing further inward movement of the plunger. Alternatively, the dose may be injected by rotating the adjustable collar 141 back to the first (or zero) position.

When the needle 181 is integrally connected to the syringe body 121, the syringe 111 is properly disposed of after an injection. When a needle hub assembly is used with the syringe body, as shown in FIGS. 2-5, the needle hub assembly can be removed and properly disposed such that the syringe may be reused. The needle hub assembly can be unthreaded from the syringe body to be disposed of. Alternatively, the first portion 122 of the syringe can be threadably connected to the second portion 124 such that the first portion 122 can be removed from the second portion 124 and the first portion can be properly disposed of. The second portion 124 of the syringe body 121, the collar 141 and the plunger 161 can then be reused.

A lens, magnifying glass or other similar device may be disposed over the window 123 in the syringe body 121 to increase the size of the dose setting indicator 145 to a user, thereby further facilitating the readability of the dose setting indicator.

Means may be provided to control rotation of the collar 141 so that the dose setting indicator 145 is centered in the window 123. Such means may also provide for rotation between dose setting indicators 145 such that collar positions between dose settings is substantially prevented. Additionally, locking means can be provided to prevent movement of the collar 141, thereby substantially preventing accidentally changing the dose setting.

Detent features can be disposed on the syringe body 121 and collar 141 to facilitate aligning the collar rotationally for the desired dose. The syringe body 121 may include at least one flexible beam that engages the detents of the collar 141. Audible and/or tactile indicia are created by overcoming the detent force to indicate proper orientation of the collar 141 when dialing a dose, thereby substantially preventing dialing a dose between dose settings. The adjustable collar 141 can have detents that accept one or more spring plungers that are assembled to the barrel of the syringe.

An indicator can be provided to indicate to a user that the plunger 161 has hit the dosage stop (seal 171 reseating in the reduced diameter end 128 of the syringe body 121), thereby substantially preventing the user from not injecting the entire desired dose. A window can provide viewing access to a mark on the plunger rod, or an audible click or other suitable means can be provided to indicate that the plunger has hit the dosage stop. Accordingly, the syringe can be provided with means to confirm dose delivery, such as, but not limited to, the dose setting indicators spinning down to zero or an audible click when the dose setting indicator reaches zero.

In an example not being part of the invention as shown in FIG. 6, a syringe 211 has a collar 241 that is completely disposed within a syringe body 221. A plunger flange 265 is disposed on a plunger rod 263 of a plunger 261. The plunger rod 263 passes through the first end 243 of the collar 241 and out through the second end 245 of the collar. The plunger flange 265 abuts the second end 245 of the collar 241. The second end 245 of the collar 241 may be recessed to substantially prevent damage from the plunger flange 265 abutting the second end. Dose setting indicators 246 are disposed on an outer surface of the collar 241 and are viewable through a window formed in the syringe body 221. Threads 249 formed on the outer surface 247 of the collar 241 engage threads 223 formed on an inner surface 225 of the syringe body.

The plunger rod 263 is keyed to the collar 241 such that rotation of the plunger rod results in rotational and axial movement of the collar, and axial movement of the plunger rod does not move the collar. The plunger rod 263 may have a substantially hexagonal shape and the collar bore may have a corresponding hexagonal shape. However, any suitable shape may be used. Rotation of the plunger rod 263 causes the collar 241 to rotate within the syringe body 221 without resulting in axial movement of the plunger 261. The plunger rod 263 can be connected to a stopper or seal 271 such that rotation of the plunger 261 does not rotate the stopper 271.

To perform an injection, the desired dose to be injected is dialed with the adjustable collar 241 by rotating the collar with respect to the syringe body 221. Initially, the seal 271 is in a first position engaging a reduced diameter portion 228 of the syringe body 221, as shown in FIG. 6. The plunger 261 is rotated, thereby rotating the collar 241 resulting in rearward axial movement of the collar 241 through the syringe body 221. When the dose setting indicator 246 corresponding to the desired dose setting appears in the window of the syringe body 221, the desired dose of liquid medicament is ready to be drawn into the syringe body 221. The larger the dose to be delivered, the further the collar 241 moves rearwardly (toward a first end 229) in the syringe body 221. The plunger 261 is pulled rearwardly through the collar 241 and syringe body 221 until the flange 265 abuts the second end 245 of the collar 241. The plunger 261 is then pushed axially back into the syringe body 221 until the seal 271 engages the reduced diameter portion 228 of the syringe body 221. When the plunger is moved axially, the keying between the plunger rod 263 and the collar 241 allows for axial movement of the plunger 261 without resulting in movement of the collar. A stop may be provided to prevent the plunger from being completely withdrawn from the syringe body 221.

The needle 281 is inserted (typically through a septum) into a container in which the liquid medication to be delivered is stored. The plunger 261 is pulled axially rearwardly, thereby moving the seal 271 rearwardly and away from the reduced diameter portion 228 of the syringe body 221. The plunger 261 is pulled axially rearwardly until the plunger flange 265 abuts the second end 245 of the collar 241, thereby limiting further movement of the plunger 261. Preferably, locking means prevents rotation of the collar 241 when the plunger 261 is being drawn outwardly while drawing in medication, thereby preventing accidental changes to the set dose. The amount of medication drawn into the syringe body 221 corresponds to the dose setting visible in the window of the syringe body 221.

Prior to drawing the dose, the plunger 261 can be moved rearwardly to draw a dose of air into the syringe body. Once the needle 281 is inserted in the container the plunger 261 is pushed axially to reseat the seal 271 in the syringe body 221, thereby dispelling the drawn air from the syringe body 221.

To inject the drawn dose, the plunger 261 is pushed axially back into the syringe body 221 until the seal 271 abuts the reduced diameter portion 228 of the syringe body 221, thereby preventing further inward movement of the plunger.

A spring may be disposed between the first end 243 of the collar 241 and the plunger handle 267 to facilitate drawing medication into the syringe body. After the collar is moved to the desired dose setting, the plunger 261 is pushed axially into the syringe body 221, thereby compressing the spring between the plunger handle 267 and the first end 243 of the collar 241. When axially pulling the plunger 261 back out of the syringe body to draw medication into the syringe body, the spring facilitates the rearward axial movement of the plunger 261.

As shown in FIG. 6, the threads 249 are disposed at opposite ends on the outer surface 247 of the collar 241. Alternatively, threads may be disposed only proximal a first end 243 of the collar 241, only proximal a second end 245 of the collar, or continuously along the outer surface 247 of the collar. Alternatively, the threads 249 may be disposed on an internal surface of the collar 241 in a similar manner.

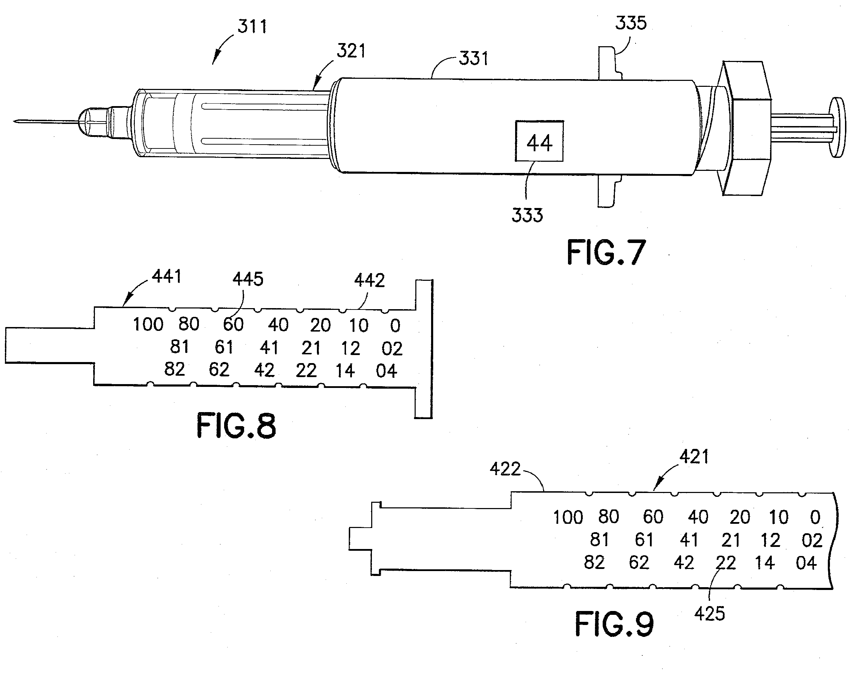

A sleeve 331, label or other suitable member may be disposed on the outer surface of the syringe body 321 having a cutout 333 therein to function as the dose setting indicator window, as shown in FIG. 7. A flange 335 may be disposed on the sleeve 331 to facilitate gripping the syringe 311 during use.

As shown in FIG. 8, a collar 441 may have the dose setting indicators 445 disposed on an outer surface 442 thereof. Alternatively, the dose setting indicators 425 are disposed on an outer surface 422 of a syringe body 421, as shown in FIG. 9.

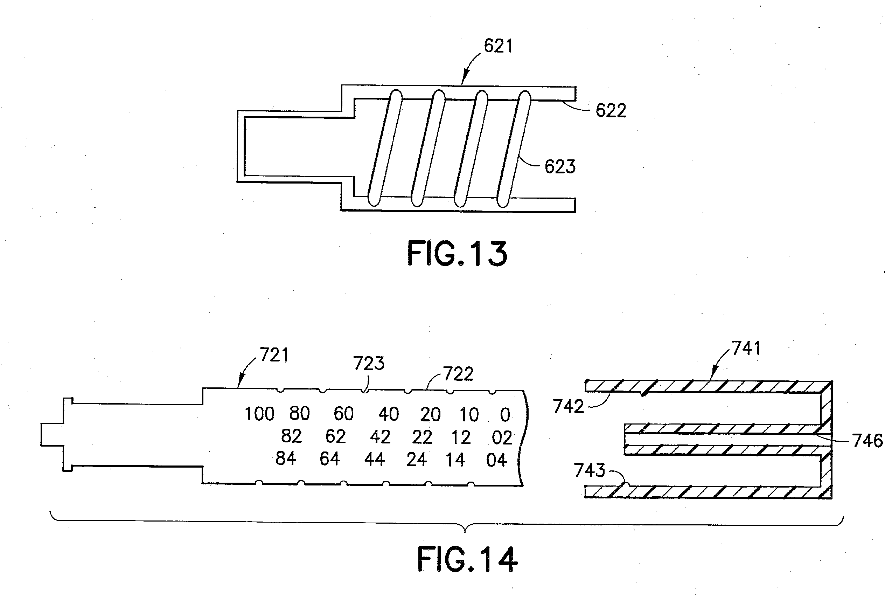

As shown in FIG. 10, a collar 541 can have a groove 543 disposed on an outer surface 542 thereof. A positive thread 523 is disposed on an inner surface 522 of a syringe body 521, as shown in FIG. 11, and receives the collar groove 543. As the handle 547 of the collar 541 is rotated, the collar translates in the syringe body 521, thereby setting the travel limit for the plunger and the allowable amount of fluid that can be drawn. Alternatively, as shown in FIGS. 12 and 13, a collar 641 can have a positive thread 643 disposed on an outer surface 642 thereof. A groove 623 is disposed on an inner surface 622 of a syringe body 621 to receive the collar thread 643.

A collar 741 can have threads 743 disposed on an inner surface 742 thereof, as shown in FIG. 14. Threads 723 are disposed on an outer surface 722 of a syringe body 721 for rotatably receiving the collar 741. A collar bore 746 is disposed within the syringe body 721 and is adapted to receive a plunger. Accordingly, by disposing the dosing knob outside at the non-patient end of the syringe body, threads on the inside of the syringe body are eliminated.

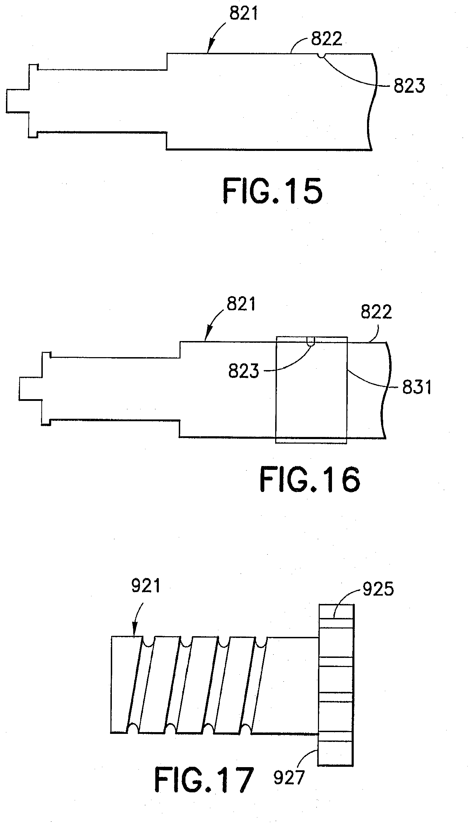

At least one protrusion 823 can be formed on an outer surface 822 of a syringe body 821, as shown in FIG. 15, for guiding a collar. The protrusion 823 can be molded into the syringe body 821. Alternatively, as shown in FIG. 16, the protrusion 823 can be formed on the outer surface 822 of the syringe body 821 by a sleeve 831 or press fit pin. The barrel may contain one or more cutouts to accept one or more plugs, each of which contains a feature at one end that is shaped to engage in the groove of the collar.

As shown in FIGS. 1, 2 and 4, the collar handle 147 can have a substantially hexagonal shape that is easily operated by a user having dexterity limitations. Alternatively, as shown in FIG. 17, a collar 921 can have a handle 927 having a plurality of ribs 925 that run substantially parallel to the rotational axis of the collar 921. The ribs 927 are easily gripped by a user having dexterity limitations. Accordingly, the collar handle 927 provides means for facilitating control of the syringe during use. Alternatively, means for facilitating control of the syringe during use can be disposed on the syringe body, such as the flange 335 disposed on the sleeve 331 on the syringe body 321 shown in FIG. 7.

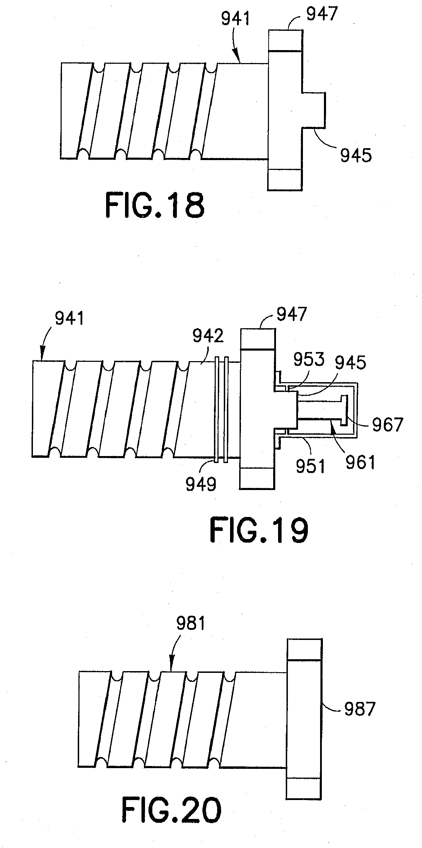

A handle 947 of a collar 941 can have a substantially cylindrical projection 945 extending outwardly therefrom, as shown in FIGS. 18 and 19. A cap 951 is received by the projection 945 to form a sterility barrier for the fluid path. The cap 951 can cover the plunger handle 967 and can have an internal ring 953 that engages the projection 945, as shown in FIG. 19, to form a sterility barrier between the plunger 961 and the collar 941. A plurality of rings 949 can be disposed on an outer surface 942 of the collar 941 to form a sterility barrier between the syringe body (such as syringe body 521 of FIG. 11) and the collar. Alternatively, when packaging for the syringe (such as syringe 111 of FIG. 1) provides the sterility barrier, a collar 981 does not have a projection extending from a handle 987.

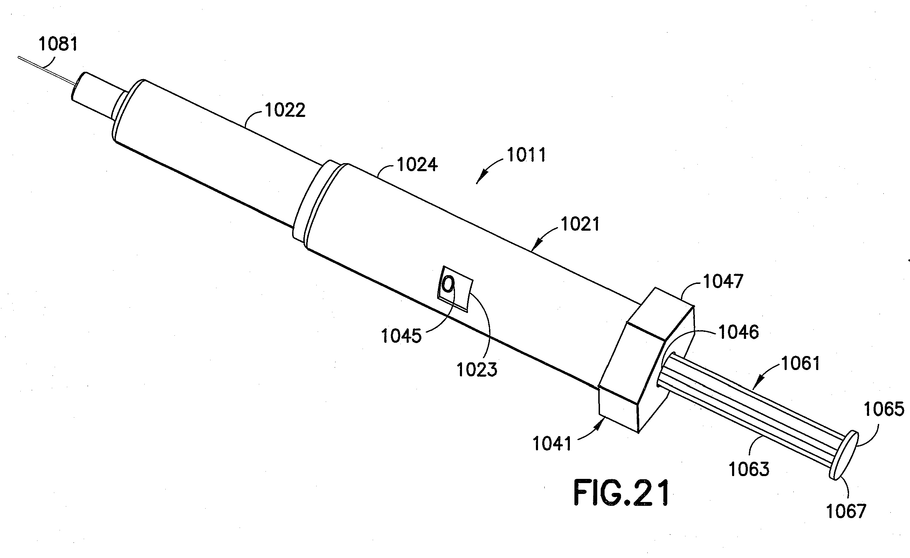

In another exemplary embodiment of the present invention, as shown in FIGS. 21 - 24, a syringe 1011 includes a syringe body 1021, a collar 141 rotationally and axially movable with respect to the syringe body 1021, and a plunger 1061 movable with respect to the collar 1041. A needle 1081 is connected to the syringe body 1021. Medication is drawn from a container through the needle 1081 into the syringe body 1021 and injected from the syringe body through the needle into an injection site. The syringe 1011 may have an integral barrel to provide a permanently attached needle or may have a threaded, snap-fit or other type of barrel design to provide a removable needle. Alternatively, the needle 1081 may be deleted altogether.

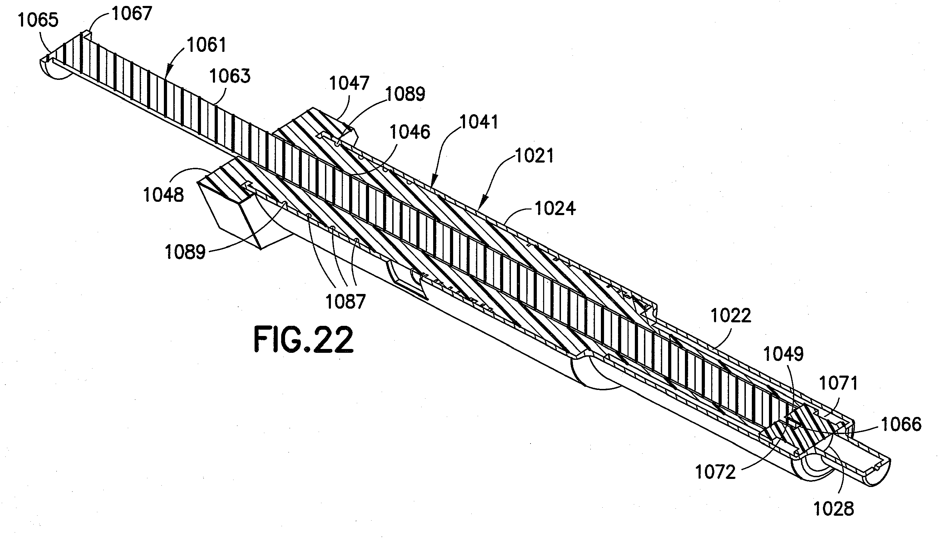

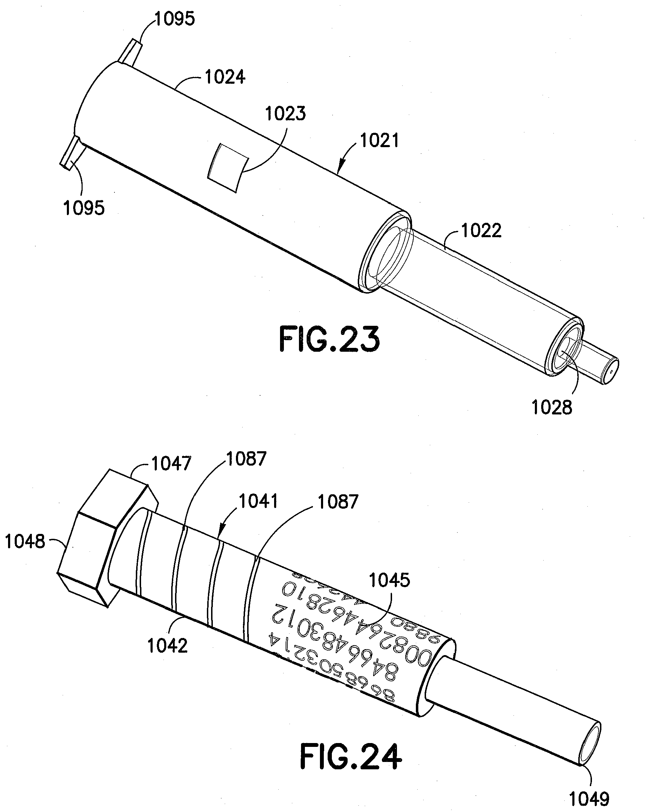

The syringe 1011 has a window 1023 through which the dose setting is displayed, preferably in large numbers that are easy for a user to see, learn and use, as shown in FIG. 21. The user dials the desired dose by rotating the collar knob 1047 with respect to the syringe body 1021. Flanges 1095 can extend outwardly from the syringe body 1021 to gripping the syringe 1011 during use. Medicine can then be drawn from a container by pulling the plunger 1061 rearwardly out of the syringe body 1021 until the plunger abuts a mechanical stop, thereby limiting the amount of medication drawn into the syringe to the set dose. As shown in FIGS. 22 and 24, rearward movement of the plunger 1061 is stopped when the stopper 1071 abuts a second end 1049 of the collar 1041.

The collar 1041 is rotationally and axially movable with respect to the syringe body 1021. Threads 1089 are disposed on an outer surface 142 of the collar 1041. A bore 1046 passes through the collar 1041 from a first end 1048 to the second end 1049 to receive the plunger rod 1063 therethrough. The outer surface 1042 of the collar 1041 has a plurality of indicators 1045, which correspond to the dose setting, disposed proximal the threads. The indicators 1045 may be in fractions of a unit, single units or multiple units. The indicators may be numerals, symbols or Braille to facilitate use by users having impairments. Alternatively, the indicators may be displayed digitally. A handle 1047 is disposed at the first end 1048 of the collar 1041 to facilitate rotation of the collar 1041 by a user to set the dose. The second end 1049 of the collar 1041 has a smaller diameter than the first end 1048 and acts as a mechanical stop for the plunger 1061 when pulling the plunger out of the collar 1041 to draw medication from a container into the syringe body 1021, thereby limiting the amount of medication drawn into the syringe to the set dose.

The collar bore 1046 can be keyed to prevent rotational movement of the plunger rod 163 with respect to the collar 1041. Accordingly, the collar 1041 is substantially prevented from accidental rotation such that a set dose is not accidentally altered by undesired rotation of the collar 1041.

The syringe body 1021 has a first portion 1022 and a second portion 1024, as shown in FIGS. 21 - 23. A diameter of the second portion 10 24 is larger than a diameter of the first portion 1022. The first portion 1022 receives medicament and the second portion receives the collar 1041, as shown in FIGS. 21 and 22. Preferably, the first and second portions are integrally formed such that the syringe body is a single, unitary member. A separate rubber stopper or seal 1071 is disposed in the first portion 1022 of the syringe body 1021, as shown in FIG. 22 to substantially prevent the second portion 1024 from contacting the fluid path. The stopper 1071 has an opening 1072 that accepts the tip of the plunger rod 1066, as shown in FIG. 22, and can be assembled and disassembled. Other configurations can be used to connect the plunger rod 1063 to the stopper 1071 to facilitate independent rotation of the plunger rod with respect to the stopper and allowing the plunger rod and stopper to move together axially. For example, a ball can be formed on the end of the plunger rod 1063 that is received in a cavity within the stopper and an opening to access the cavity is smaller than the stopper opening, thereby substantially preventing the plunger rod from being withdrawn from the stopper.

The first portion 1022 of the syringe body 1021 is preferably transparent so that the medicine is visible to the user and following an injection the user can verify that the medicine has been injected. The second portion 1024 of the syringe body 1021 has a window 1023 that exposes the desired dose setting.

Internal threads 1087 are disposed on an inner surface of the second portion 1024 of the syringe body 1021, and engage the threads on the outer surface 1042 of the collar 1041 for rotational and axial movement of the collar with respect to the syringe body 1021. The window 1023 in the second portion 1024 of the syringe body 1021 allows a user to view the dose setting indicators 1045 as the collar 1041 is rotated out of the syringe body 1021. The dose setting indicator 1045 viewable in the window 1023 corresponds to the dose setting.

The plunger 1061 includes a plunger rod 1063 axially movable with respect to the collar 1041. A first end 1065 of the plunger rod 1063 has a plunger head 1067 disposed externally of the collar 1041. The plunger head 1067 is larger than the collar bore 1046 to prevent the first end 1065 of the plunger from entering the bore. A second end 1066 of the plunger rod 1063 is disposed externally of the collar 1041 and within the stopper 1071 in the syringe body 1021.

The stopper, or seal, 1071 is connected to the second end 1066 of the plunger rod 1063. The stopper 1071 is movable within the syringe body 1021 between a first position at a reduced diameter portion 1028 of the syringe body and a second position at the mechanical stop at the second end 1049 of the collar 1041. The stopper 1071 creates a seal within the syringe body 1021 that prevents medication from passing beyond the seal.

To perform an injection, the desired dose to be injected is dialed in with the adjustable collar 1041. Initially, the stopper 1071 is in the first position engaging the reduced diameter portion 1028 of the syringe body, as shown in FIG. 22. The collar handle 1047 is rotated, thereby axially moving the collar 1041 out of the syringe body 1021. When the dose indicator 1045 corresponding to the desired dose setting appears in the window 1023, the desired dose is ready to be drawn into the first portion 1022 of the syringe body 1021. The larger the dose to be delivered, the further the collar 1041 moves out of the syringe body 1021 and the further from the reduced diameter portion 1028 of the syringe body 1021 the second end 1049 of the collar 1041 moves.

The needle 1081 is inserted (typically through a septum) into a container, such as an insulin vial, in which the liquid medication to be delivered is stored. The plunger head 1067 is drawn out of the collar 1041, thereby moving the stopper 1071 rearwardly, until the stopper 1071 abuts the second end 1049 of the collar 1041, thereby limiting further movement of the plunger rod 1063. The amount of medication drawn into the syringe body 1021 corresponds to the dose setting visible in the window 1023.

To inject the drawn dose, the plunger 1061 is pushed back into the collar 1041 until the seal 1071 returns to the first position abutting the reduced diameter portion 1028 of the syringe body 1021, thereby preventing further inward movement of the plunger rod 1063.

A syringe can be provided having a hollow threaded post through which the plunger passes. The threaded handle has a dial handle disposed at the end thereof. The threaded post is secured to the barrel such that the threaded post can only rotate with respect to the barrel and axial movement of the threaded post is substantially prevented. A collar, which is not free to rotate by means of a keyed feature and slot in the barrel, travels on the threaded post. As the dial handle and threaded post rotate, the collar translates up and down the threaded post to serve as a stop for the plunger.

A two-piece cap can be used to provide sterility. A first piece of the cap is removable. The second piece of the cap remains on the syringe and has a notch that serves as a window for the dose setting.

A notch can be formed in the syringe body to function as a window for viewing the dose setting.

A plurality of small bumps can be provided on the syringe body to facilitate press-fitting a needle assembly to the syringe body.

The syringe housing can have scale markings thereon indicating the amount of liquid medicine for reference purposes.

The syringe body can include a threaded member integrated with the syringe housing during manufacturing to provide the threaded feature. The seal can be assembled into the syringe body at the patient (needle) end prior to the needle assembly being integrated with the syringe housing and the plunger can be assembled from the opposite end of the syringe housing. Alternatively, the seal can be assembled from the end of the syringe housing opposite the patient (needle) end.

A prefilled liquid medicine cartridge can be loaded into the syringe housing.

Two adjustable knobs can be provided on the syringe. The first knob adjusts the 10's digit of the dose setting, i.e., dialing the dose to discrete locations at 10, 20, 30 and so forth. The second knob adjusts the singles digit and turns within or around the first knob. This arrangement maintains a compact diameter of the syringe and allows for the dose setting units to range from 1 to 100. The first and/or second knobs can also translate rather than rotating on a helix, and can click into preset locations along the syringe body.

The adjustable knob can have a movable or detachable component that engages with the back of the seal when the member contacts the seal during aspiration. The component can be connected by a snap-fit, magnetically or in any other suitable manner. When the plunger is depressed, the component moves with the seal, thereby providing the user with a visual indication that the plunger was fully pulled back.

One or more tracks having detents can be used to facilitate obtaining the desired dosage.

The syringe can accommodate needles of various lengths and gauge, including, but not limited to, micro-needles. Alternatively, the syringe may be used without a needle, as for example to administer a fluid through an intravenous line via a needleless valve, port or injection site.

The syringe can be made of various materials, such as, but not limited to, plastic or glass. The syringe can be made in any color. The barrel and stopper materials of the syringe can be made of materials that allow the drawn medication to be stored in the syringe for future medication delivery without causing any degradation or loss of potency of the medication.

The syringe can have safety features to prevent accidental needle sticks and/or reuse of the needle. The syringe can have an auto-disable feature to prevent reuse.

The syringe can include a feature to facilitate dialing and drawing a first dose from a first container and then dialing and drawing a dose from a second container to support medication mixing, such as insulin mixing.

The scale of the dose setting indicators can be a function of the intended use of the syringe, such as, but not limited to, U-100, U-40 and mL. Alternatively, the dose scale can be based on patient weight. The scale is the patient's weight, such that the syringe calculates the volume and dosage instead of the health care professional. Certain characteristics of the syringe can vary by total dosage and the chosen scale, such as the thread pitch, thread length and diameters. The syringe can also hold varying nominal volumes depending on the intended application.

A threaded fitting can be disposed at the needle end of the syringe to accept a pen needle. The dose setting indicators can be in the form of an odometer that is driven by the plunger.

A prefilled single-use syringe can accommodate various needle lengths, including 4 mm, and has a depth stop. The plunger stop is on the side of the adjustable collar farthest from the needle. The plunger starts in the most retracted position and when the plunger is depressed, the plunger can only travel until it abuts against the adjustable collar set to the desired dose. After the dose has been delivered, the syringe can be discarded. The capacity of the syringe can vary depending on the use. For example, the syringe can be sized to contain 10, 20, 30 or 50 units, but the size of the syringe is not limited to these examples.

Alternatively, the syringe can include a liquid medication, such as insulin, a filled reservoir and a dial dose. A valve plunger is used to dispense the desired amount of medication and a pressurized gas is used to provide the appropriate activation. Alternatively, a flapper valve can be used to regulate the reservoir to allow for the appropriate amount of medication to be delivered. Alternatively, the reservoir can be divided into two chambers (usable and non-usable chambers) by a seal or other suitable means. A fluid connecting path connects the two chambers. The amount of medication is moved from the usable chamber to the non-usable chamber so that only the appropriate amount of medication is delivered.

A syringe according to any of the exemplary embodiments can be prefilled or filled by a user. A prefilled syringe can have a plunger stop disposed on the side of the adjustable collar farthest from the needle. The plunger starts in the most retracted position. When the plunger is depressed, it can only travel until it abuts the adjustable collar set to the desired dose. Alternatively, instead of being discarded after a single use, the adjustable collar can be reset to deliver a subsequent dose. This can be achieved by the use of double cams or other suitable means. The syringe can include a septum and threads that accept a pen needle.

A syringe according to any of the exemplary embodiments can be filled to accommodate multiple doses. The adjustable collar can be labeled to reflect set doses during the day. For example, the collar can be labeled with the numerals 1, 2 and 3 (or A, B and C; or AM, PM, MT and NT to reflect meal time or night time; or any other suitable arrangement). There may also be a line on the adjustable collar that becomes wider in thickness as the collar rotates to let the user know that they are moving from one setting to another. A seal can be provided on the adjustable collar to ensure that the fluid path remains sterile as the collar translates in the barrel.

A syringe according to any of the exemplary embodiments can include a feature that facilitates expelling air bubbles after drawing in the medication.

The syringe can be adapted for use with dry, powdered or lyophilized medications, in addition to or in lieu of liquid medications.

The foregoing embodiments and advantages are merely exemplary and are not to be construed as limiting the scope of the present invention as defined by the appended claims. The description of exemplary embodiments of the present invention is intended to be illustrative, and not to limit the scope of the present invention.

## Claims

1. A syringe, comprising:
a body (121, 1021) having a first portion (122, 1022) and a second portion (124, 1024), said first portion (122, 1022) receiving a medicament, said first portion (122, 1022) having a first diameter and said second portion (124, 1024) having a second diameter, said first diameter being smaller than said second diameter;
a collar (141, 1041) rotatably disposed in said body (121, 1021), said collar (141, 1041) being rotatable to set a desired dose of medication drawn into said first portion (122, 1022);
a plunger (161, 1061) movably connected to said collar (141, 1041), said plunger (161, 1061) having a plunger rod (163, 1063); and
a stopper (171, 1071) disposed at an end of said plunger rod (163, 1063) to facilitate drawing in and dispensing the medicament,
wherein
said collar (141, 1041) has a first end (148, 1048), a second end (149, 1049), and a bore (146, 1046) passing through said collar (141, 1041) from said first end (148, 1048) to said second end (149, 1049) to receive said plunger rod (163, 1063) therethrough,
wherein
an outer surface (142, 1042) of said collar (141, 1041) has a threaded portion to engage a corresponding threaded portion on an inner surface of said second portion (124, 1024) of said body,
**characterized in that** said second end (149, 1049) of said collar (141, 1041) has a smaller diameter than said threaded portion of said collar (141, 1041), said second end (149, 1049) of said collar (141, 1041) being disposed in said first portion of said body (121, 1021),
said second end (149, 1049) acting as a mechanical stop for said plunger (161, 1061) by abutting said stopper (171, 1071) when pulling said plunger (161, 1061) out of said collar (141, 1041), thereby limiting the medication drawn into said body to said set desired dose.

2. The syringe according to claim 1, wherein
said stopper (171, 1071) and plunger rod (163, 1063) are unitarily formed as a single piece.

3. The syringe according to claim 1, wherein
said plunger rod (163, 1063) and said stopper (171, 1071) are separately formed as two pieces.

4. The syringe according to claim 1, 2, or 3, wherein
rotation of said plunger rod (163, 1063) rotates said collar (141, 1041) and wherein axial movement of said plunger rod (163, 1063) does not result in movement of said collar (141, 1041).

5. The syringe according to claim 1, 2, 3, or 4, wherein
said outer surface (142, 1042) of said collar (141, 1041) having said threaded portion is disposed outside of said first portion (122, 1022) of said body (121, 1021).

6. The syringe according to claim 1, 2, 3, 4, or 5, wherein
said collar (141, 1041) comprises a handle (147, 1047) disposed at said first end (148, 1048) of said collar (141, 1041) to facilitate rotation of said collar (141, 1041) to set said desired dose.

7. The syringe according to claim 1, 2, 3, 4, 5, or 6, wherein said first portion (122, 1022) of said body (121, 1021) and said second portion (124, 1024) of said body (121, 1021) are integrally formed.

8. The syringe according to claim 1, 2, 3, 4, 5, 6, or 7, wherein said first end (148, 1048) of said collar (141, 1041) has a diameter larger than said diameter of said threaded portion of said collar (141, 1041).

9. The syringe according to claim 1, 2, 3, 4, 5, 6, 7, or 8, wherein
said first portion (122, 1022) of said body (121, 1021) has a first end in communication with said second portion (124, 1024) of said body (121, 1021) and a second end opposite said first end,
said plunger (161, 1061) is movable between a first and a second position, when said plunger (161, 1061) is in said first position said stopper is disposed in said first portion of said body abutting inner said second end of said first portion of said body, and when said plunger is in said second position said stopper is disposed in said first portion of said body abutting said second end of said collar.

10. The syringe according to any one of claims 1, 2, 3, 4, 5, 6, 7, 8, or 9, wherein a needle is connected to said first portion of said body to facilitate drawing in and dispensing of the medicament.

11. The syringe according to claim 10, wherein
a shield is connected to said body to cover said needle.

## Patentansprüche

1. Spritze, die aufweist:
einen Körper (121, 1021) mit einem ersten Abschnitt (122, 1022) und einem zweiten Abschnitt (124, 1024), wobei der erste Abschnitt (122, 1022) ein Medikament aufnimmt, wobei der erste Abschnitt (122, 1022) einen ersten Durchmesser aufweist und der zweite Abschnitt (124, 1024) einen zweiten Durchmesser aufweist, wobei der erste Durchmesser kleiner als der zweite Durchmesser ist;
eine Manschette (141, 1041), die drehbar in dem Körper (121, 1021) angeordnet ist, wobei die Manschette (141, 1041) drehbar ist, um eine gewünschte Dosis eines Medikaments einzustellen, die in den ersten Abschnitt (122, 1022) eingezogen wird;
einen Kolben (161, 1061), der bewegbar mit der Manschette (141, 1041) verbunden ist, wobei der Kolben (161, 1061) eine Kolbenstange (163, 1063) aufweist; und
einen Stopper (171, 1071), der an einem Ende der Kolbenstange (163, 1063) angeordnet ist, um das Einziehen und Ausgeben des Medikaments zu vereinfachen,
wobei die Manschette (141, 1041) ein erstes Ende (148, 1048), ein zweites Ende (149, 1049) und eine Bohrung (146, 1046) aufweist, die durch die Manschette (141, 1041) von dem ersten Ende (148, 1048) zu dem zweiten Ende (149, 1049) verläuft, um die Kolbenstange (163, 1063) dort hindurch aufzunehmen,
wobei eine Außenfläche (142, 1042) der Manschette (141, 1041) einen Gewindeabschnitt aufweist, um mit einem entsprechenden Gewindeabschnitt auf einer Innenfläche des zweiten Abschnitts (124, 1024) des Körpers zusammenzugreifen,
**dadurch gekennzeichnet, dass**
das zweite Ende (149, 1049) der Manschette (141, 1041) einen kleineren Durchmesser als der Gewindeabschnitt der Manschette (141, 1041) aufweist,
wobei das zweite Ende (149, 1049) der Manschette (141, 1041) in dem ersten Abschnitt des Körpers (121, 1021) angeordnet ist,
wobei das zweite Ende (149, 1049) als ein mechanischer Anschlag für den Kolben (161, 1061) wirkt, indem er beim Herausziehen des Kolbens (161, 1061) aus der Manschette (141, 1041) an dem Stopper (171, 1071) anliegt, wodurch das in den Körper eingezogene Medikament auf die eingestellte gewünschte Dosis begrenzt wird.

2. Spritze nach Anspruch 1, wobei der Stopper (171, 1071) und die Kolbenstange (163, 1063) einheitlich als ein einziges Teil ausgebildet sind.

3. Spritze nach Anspruch 1, wobei die Kolbenstange (163, 1063) und der Stopper (171, 1071) separat als zwei Teile ausgebildet sind.

4. Spritze nach Anspruch 1, 2 oder 3, wobei die Drehung der Kolbenstange (163, 1063) die Manschette (141, 1041) dreht, und wobei die axiale Bewegung der Kolbenstange (163, 1063) nicht in einer Bewegung der Manschette (141, 1041) resultiert.

5. Spritze nach Anspruch 1, 2, 3 oder 4, wobei die Außenfläche (142, 1042) der Manschette (141, 1041) mit dem Gewindeabschnitt außerhalb des ersten Abschnitts (122, 1022) des Körpers (121, 1021) angeordnet ist.

6. Spritze nach Anspruch 1, 2, 3, 4 oder 5, wobei die Manschette (141, 1041) einen Griff (147, 1047) aufweist, der an dem ersten Ende (148, 1048) der Manschette (141, 1041) angeordnet ist, um die Drehung der Manschette (141, 1041) zum Einstellen der gewünschten Dosis zu vereinfachen.

7. Spritze nach Anspruch 1, 2, 3, 4, 5 oder 6, wobei der erste Abschnitt (122, 1022) des Körpers (121, 1021) und der zweite Abschnitt (124, 1024) des Körpers (121, 1021) einstückig ausgebildet sind.

8. Spritze nach Anspruch 1, 2, 3, 4, 5, 6 oder 7, wobei das erste Ende (148, 1048) der Manschette (141, 1041) einen Durchmesser aufweist, der größer als der Durchmesser des Gewindeabschnitts der Manschette (141, 1041) ist.

9. Spritze nach Anspruch 1, 2, 3, 4, 5, 6, 7 oder 8, wobei der erste Abschnitt (122, 1022) des Körpers (121, 1021) ein erstes Ende, das in Verbindung mit dem zweiten Abschnitt (124, 1024) des Körpers (121, 1021) steht, und ein zweites Ende entgegengesetzt zu dem ersten Ende aufweist,
wobei der Kolben (161, 1061) zwischen einer ersten und einer zweiten Position bewegbar ist,
wobei, wenn der Kolben (161, 1061) in der ersten Position ist, der Stopper in dem ersten Abschnitt des Körpers angeordnet ist und am zweiten Ende des ersten Abschnitts des Körpers anliegt, und wenn der Kolben in der zweiten Position ist, der Stopper in dem ersten Abschnitt des Körpers angeordnet ist und an dem zweiten Ende der Manschette anliegt.

10. Spritze nach einem der Ansprüche 1, 2, 3, 4, 5, 6, 7, 8 oder 9, wobei eine Nadel mit dem ersten Abschnitt des Körpers verbunden ist, um das Einziehen und Ausgeben des Medikaments zu vereinfachen.

11. Spritze nach Anspruch 10, wobei eine Abschirmung mit dem Körper verbunden ist, um die Nadel zu bedecken.

## Revendications

1. Seringue, comprenant :
un corps (121, 1021) ayant une première partie (122, 1022) et une deuxième partie (124, 1024), ladite première partie (122, 1022) recevant un médicament, ladite première partie (122, 1022) ayant un premier diamètre et ladite deuxième partie (124, 1024) ayant un deuxième diamètre, ledit premier diamètre étant inférieur audit deuxième diamètre ;
un collier (141, 1041) disposé de manière rotative dans ledit corps (121, 1021), ledit collier (141, 1041) pouvant tourner pour régler une dose souhaitée de médicament aspiré dans ladite première partie (122, 1022) ;
un piston (161, 1061) relié de manière mobile audit collier (141, 1041), ledit piston (161, 1061) ayant une tige de piston (163, 1063) ; et
un bouchon (171, 1071) disposé au niveau d'une extrémité de ladite tige de piston (163, 1063) pour faciliter l'aspiration et la distribution du médicament,
dans laquelle
ledit collier (141, 1041) a une première extrémité (148, 1048), une deuxième extrémité (149, 1049), et un alésage (146, 1046) passant à travers ledit collier (141, 1041) de ladite première extrémité (148, 1048) à ladite deuxième extrémité (149, 1049) pour recevoir ladite tige de piston (163, 1063) à travers celui-ci,
dans laquelle
une surface externe (142, 1042) dudit collier (141, 1041) a une partie filetée pour s'engager avec une partie filetée correspondante sur une surface interne de ladite deuxième partie (124, 1024) dudit corps,
**caractérisée en ce que** ladite deuxième extrémité (149, 1049) dudit collier (141, 1041) a un diamètre inférieur à celui de ladite partie filetée dudit collier (141, 1041), ladite deuxième extrémité (149, 1049) dudit collier (141, 1041) étant disposée dans ladite première partie dudit corps (121, 1021),
ladite deuxième extrémité (149, 1049) agissant comme une butée mécanique pour ledit piston (161, 1061) en venant en butée contre ledit bouchon (171, 1071) lors de la traction dudit piston (161, 1061) hors dudit collier (141, 1041), limitant ainsi le médicament aspiré dans ledit corps à ladite dose souhaitée réglée.

2. Seringue selon la revendication 1, dans laquelle
ledit bouchon (171, 1071) et la tige de piston (163, 1063) sont formés unitairement en une seule pièce.

3. Seringue selon la revendication 1, dans laquelle
ladite tige de piston (163, 1063) et ledit bouchon (171, 1071) sont formés séparément en deux pièces.

4. Seringue selon la revendication 1, 2 ou 3, dans laquelle
la rotation de ladite tige de piston (163, 1063) fait tourner ledit collier (141, 1041) et dans laquelle le déplacement axial de ladite tige de piston (163, 1063) n'entraîne pas le déplacement dudit collier (141, 1041).

5. Seringue selon la revendication 1, 2, 3 ou 4, dans laquelle
ladite surface externe (142, 1042) dudit collier (141, 1041) ayant ladite partie filetée est disposée à l'extérieur de ladite première partie (122, 1022) dudit corps (121, 1021).

6. Seringue selon la revendication 1, 2, 3, 4 ou 5, dans laquelle
ledit collier (141, 1041) comprend une poignée (147, 1047) disposée au niveau de ladite première extrémité (148, 1048) dudit collier (141, 1041) pour faciliter la rotation dudit collier (141, 1041) pour régler ladite dose souhaitée.

7. Seringue selon la revendication 1, 2, 3, 4, 5 ou 6, dans laquelle
ladite première partie (122, 1022) dudit corps (121, 1021) et ladite deuxième partie (124, 1024) dudit corps (121, 1021) sont formées d'un seul tenant.

8. Seringue selon la revendication 1, 2, 3, 4, 5, 6 ou 7, dans laquelle
ladite première extrémité (148, 1048) dudit collier (141, 1041) a un diamètre supérieur audit diamètre de ladite partie filetée dudit collier (141, 1041).

9. Seringue selon la revendication 1, 2, 3, 4, 5, 6, 7 ou 8, dans laquelle
ladite première partie (122, 1022) dudit corps (121, 1021) a une première extrémité en communication avec ladite deuxième partie (124, 1024) dudit corps (121, 1021) et une deuxième extrémité opposée à ladite première extrémité,
ledit piston (161, 1061) est mobile entre une première et une deuxième positions, lorsque ledit piston (161, 1061) est dans ladite première position, ledit bouchon est disposé dans ladite première partie dudit corps en butée contre ladite deuxième extrémité de ladite première partie dudit corps, et lorsque ledit piston est dans ladite deuxième position, ledit bouchon est disposé dans ladite première partie dudit corps en butée contre ladite deuxième extrémité dudit collier.

10. Seringue selon l'une quelconque des revendications 1, 2, 3, 4, 5, 6, 7, 8 ou 9, dans laquelle
une aiguille est reliée à ladite première partie dudit corps pour faciliter l'aspiration et la distribution du médicament.

11. Seringue selon la revendication 10, dans laquelle
une protection est reliée audit corps pour couvrir ladite aiguille.
